# EUROPEAN PATENT APPLICATION

(11) **EP 3 932 439 A1**
(43) Date of publication of application: **05.01.2022**
(21) Application number: 20776921.7
(22) Date of filing: 27.03.2020
(51) Int. Cl.: A61L 31/04, A61L 31/06, A61L 31/14, A61B 17/11

(54) **MEDICAL DEVICE**

(30) Priority: 28.03.2019 JP 2019065058
(71) Applicant: TERUMO Kabushiki Kaisha, Tokyo 151-0072 (JP)
(72) Inventor: ARAMAKI, Naoki, Ashigarakami-gun, Kanagawa 259-0151 (JP); KAI, Miho, Ashigarakami-gun, Kanagawa 259-0151 (JP); FUJII, Anri, Ashigarakami-gun, Kanagawa 259-0151 (JP); UCHITOMI, Kensuke, Ashigarakami-gun, Kanagawa 259-0151 (JP)
(74) Representative: Casalonga
(86) International application number: PCT/JP2020/014298
(87) International publication number: WO 2020/196887

(57) **Abstract**

[Object] There is provided a medical device capable of reducing risk factors of an anastomotic leakage after a surgical operation is performed.

[Means for Solution] An adhesion promoting device 100 includes an adhesion promotion sheet 110 provided with an adhesion promotion portion 110A promoting adhesion of biological tissues, and a holding portion 120 that allows the adhesion promotion sheet 110 to be hooked and held on a biological organ to be joined.

## Description

### Technical Field

The present invention relates to a medical device.

### Background Art

In a medical field, a medical procedure (for example, anastomosis for a digestive tract) of joining biological organs to each other by performing a surgical operation is known. In a case where the medical procedure as described above is performed, as a prognosis determinant after surgery, a fact is important that there is no delay in adhesion in a joint portion joined between the biological organs.

In the medical procedure of joining the biological organs, various methods and various medical instruments are used. For example, a method of suturing the biological organs by using a biodegradable suture, or a method of using a mechanical joint device (refer to PTL 1) for performing anastomosis by using a stapler has been proposed. In particular, in a case where anastomosis is performed using the mechanical joint device, compared to a method of using the suture, a joining force between the biological organs can be improved in the joint portion. Accordingly, risk factors of an anastomotic leakage can be reduced.

### Citation List

### Patent Literature

PTL 1: JP-T-2007-505708

### Summary of Invention

### Technical Problem

However, a progress degree of the adhesion in the joint portion depends on a state of biological tissues in a joint object site (joint target site) of a patient. Therefore, for example, even in a case where the joint device as disclosed in PTL 1 is used, depending on the state of the biological tissues of the patient, there is a possibility that the risk factors of the anastomotic leakage cannot be sufficiently reduced.

Therefore, the present invention aims to provide a medical device capable of reducing risk factors of an anastomotic leakage after a surgical operation is performed.

### Solution to Problem

According to an embodiment of the present invention, there is provided a medical device including an adhesion promotion sheet configured to include an adhesion promotion portion promoting adhesion of biological tissues, and a holding portion configured to hook and hold the adhesion promotion sheet on a biological organ to be joined.

### Advantageous Effect of Invention

According to the medical device according to the present invention, the adhesion of the biological tissues of the biological organs can be promoted by interposing the adhesion promotion sheet between the joint target sites of the biological organs to be joined. In addition, the operator can hook and hold the adhesion promotion sheet on a portion of the biological organs to be joined by the holding portion. Therefore, the operator can prevent the adhesion promotion sheet from falling off from the biological organs during a medical procedure. Therefore, the risk of anastomotic leakage of the biological organs can be effectively reduced.

### Brief Description of Drawings

[Fig. 1A] Fig. 1A is a perspective view illustrating a form of a medical device of the present invention.
[Fig. 1B] Fig. 1B is a diagram for describing a usage example of the medical device in Fig. 1A.
[Fig. 2] Fig. 2 is an enlarged cross-sectional view illustrating a portion of a cross section taken along line 2A-2A in Fig. 1A.
[Fig. 3A] Fig. 3A is a perspective view illustrating Modification Example 1 of the medical device of the present invention.
[Fig. 3B] Fig. 3B is a diagram for describing a usage example of the medical device in Fig. 3A.
[Fig. 4A] Fig. 4A is a perspective view illustrating Modification Example 2 of the medical device of the present invention.
[Fig. 4B] Fig. 4B is a diagram for describing a usage example of the medical device in Fig. 4A.
[Fig. 5] Fig. 5 is a diagram for describing Modification Example 3 of the medical device of the present invention.
[Fig. 6] Fig. 6 is a flowchart illustrating each procedure of a treatment method using the medical device.
[Fig. 7] Fig. 7 is a flowchart illustrating a procedure of an embodiment of the treatment method (pancreatic parenchyma-jejunum anastomosis).
[Fig. 8] Fig. 8 is a schematic perspective view for describing the pancreatic parenchyma-jejunum anastomosis.
[Fig. 9] Fig. 9 is a schematic perspective view for describing the pancreatic parenchyma-jejunum anastomosis.
[Fig. 10] Fig. 10 is a schematic perspective view for describing the pancreatic parenchyma-jejunum anastomosis.
[Fig. 11] Fig. 11 is a schematic perspective view for describing the pancreatic parenchyma-jejunum anastomosis.
[Fig. 12] Fig. 12 is a schematic cross-sectional view for describing the pancreatic parenchyma-jejunum anastomosis.
[Fig. 13] Fig. 13 is a schematic perspective view for describing the pancreatic parenchyma-jejunum anastomosis.
[Fig. 14] Fig. 14 is a schematic perspective view for describing the pancreatic parenchyma-jejunum anastomosis.
[Fig. 15] Fig. 15 is a schematic perspective view for describing the pancreatic parenchyma-jejunum anastomosis.

### Description of Embodiments

Hereinafter, embodiments of the present invention will be described with reference to the attached drawings. In the description of the drawings, the same elements are designated by the same reference numerals, and duplicate description will be omitted. In addition, dimensional proportions in the drawings are exaggerated and different from actual proportions for convenience of description, in some cases.

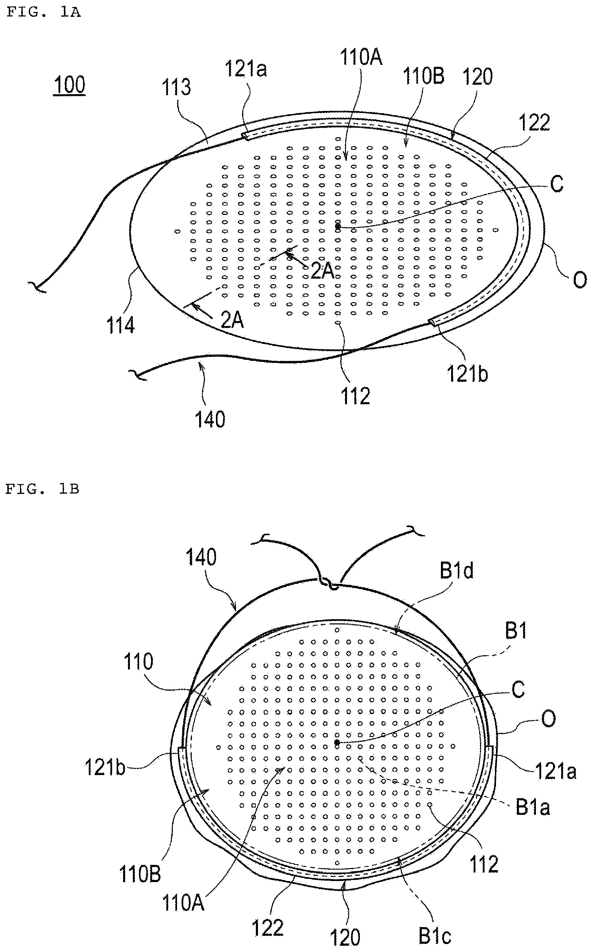

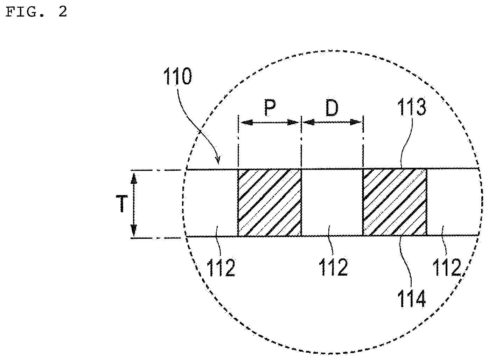

Fig. 1A is a perspective view illustrating a form of a medical device 100. Fig. 1B is a diagram illustrating a usage example of the medical device 100 in Fig. 1A, and is a view of a cut surface B1a of a pancreatic parenchyma B1 viewed from a front. Fig. 2 is an enlarged cross-sectional view illustrating a portion of the cross section taken along line 2A-2A in Fig. 1A.

### <Medical Device 100>

As illustrated in Fig. 1A, the medical device 100 includes an adhesion promotion sheet 110 disposed between biological organs to be joined, and a holding portion 120 that allows the adhesion promotion sheet 110 to be hooked and held on a biological organ to be joined (pancreatic parenchyma B1).

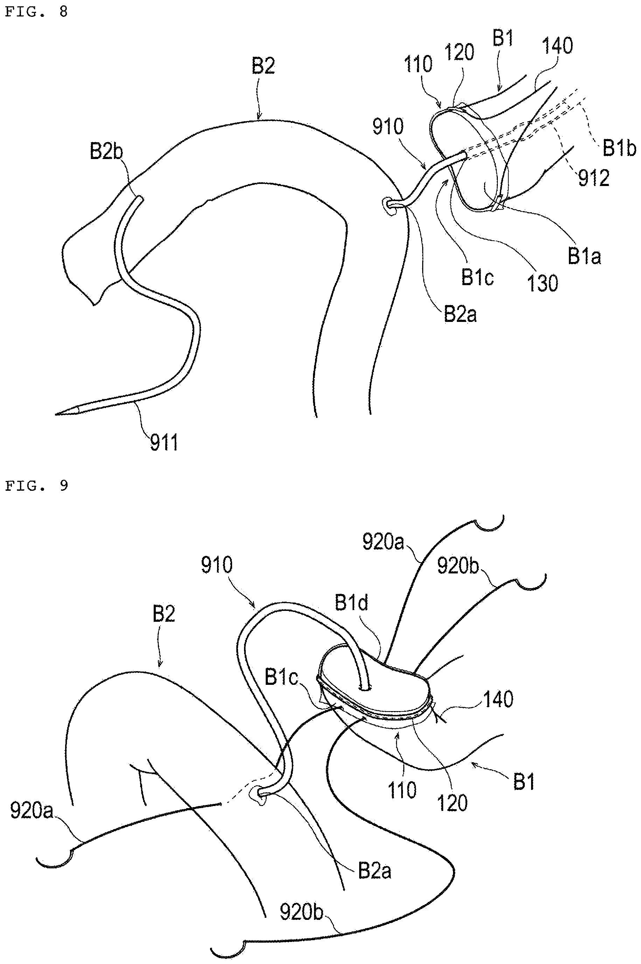

As illustrated in Figs. 8 to 15, the medical device 100 can be applied to a medical procedure of joining predetermined biological organs (for example, anastomosis for a digestive tract) . As will be described later, in the description of the present specification, pancreatic parenchyma-jejunum anastomosis will be described as an example of the medical procedure using the medical device 100.

<Adhesion Promotion Sheet 110>

As illustrated in Fig. 1A, the adhesion promotion sheet 110 includes an adhesion promotion portion 110A that promotes adhesion of biological tissues formed from a biodegradable sheet having a plurality of through-holes 112. The adhesion promotion portion 110A is formed in a predetermined range including a central portion C in a plane direction of the adhesion promotion sheet 110.

The adhesion promotion sheet 110 has a frame portion 110B provided more outside the adhesion promotion sheet 110 than the adhesion promotion portion 110A in the plane direction. The frame portion 110B is formed in a certain range including an outer peripheral edge O of the adhesion promotion sheet 110 so as to surround the periphery of the adhesion promotion portion 110A. In the present embodiment, the through-hole 112 is not formed in the frame portion 110B.

### <Adhesion Promotion Portion 110A >

As illustrated in Fig. 1A, the through-holes 112 formed in the adhesion promotion portion 110A are regularly and periodically provided in the plane direction of the adhesion promotion sheet 110. However, each through-hole 112 may be randomly provided at each portion in the plane direction of the adhesion promotion sheet 110.

As illustrated in Fig. 2, each through-hole 112 extends substantially vertically between a front surface 113 and a rear surface 114 along the thickness direction of the adhesion promotion sheet 110 (vertical direction in Fig. 2). Each through-hole 112 may be bent or curved in a zigzag shape between the front surface 113 and the rear surface 114 in a cross section along the thickness direction of the adhesion promotion sheet 110.

Each through-hole 112 has a substantially circular planar shape (shape when the front surface 113 of the adhesion promotion sheet 110 or the rear surface 114 of the adhesion promotion sheet 110 is viewed in a plan view). However, the planar shape of each through-hole 112 is not particularly limited, and may be, for example, an ellipse or a polygon (rectangle or triangle) . In addition, the plane shape and the cross-sectional shape may be different for each through-hole 112.

The adhesion promotion sheet 110 has a substantially circular planar shape. However, the planar shape of the adhesion promotion sheet 110 is not particularly limited, and may be, for example, an ellipse or a polygon (rectangle or triangle).

The thickness of the adhesion promotion sheet 110 (dimension T illustrated in Fig. 2) is not particularly limited, and is preferably 0.05 to 0.3 mm, more preferably 0.1 to 0.2 mm. In a case where the thickness of the adhesion promotion sheet 110 is 0.05 mm or more (particularly in a case of 0.1 mm or more), the adhesion promotion portion 110A can be provided with such strength that the adhesion promotion portion 110A is not damaged when the adhesion promotion sheet 110 is handled. On the other hand, in a case where the thickness of the adhesion promotion sheet 110 is 0.3 mm or less (particularly in a case of 0.2 mm or less), the adhesion promotion portion 110A can be in close contact with the biological tissue to which the adhesion promotion sheet 110 is applied and can be provided with sufficient flexibility to follow the biological tissue.

In the adhesion promotion portion 110A, a ratio value of the hole diameter D (distance D illustrated in Fig. 2) of the through-hole 112 to the pitch P (distance P illustrated in Fig. 2 and the distance between the through-holes 112 adjacent to each other) of the through-hole 112 is preferably 0.25 or more and less than 40. In a case where the planar shape of the through-hole 112 is a perfect circle, the hole diameter D of the through-hole 112 is equal to the diameter of the perfect circle. On the other hand, in a case where the planar shape of the through-hole 112 is not a perfect circle, the diameter of a perfect circle (equivalent circle diameter) having the same area as an area of an opening portion of the through-hole 112 (portion of the through-hole 112 facing the front surface 113 or the rear surface 114) can be defined as the hole diameter D of the through-hole 112.

Since the adhesion promotion portion 110A includes a plurality of through-holes 112, there are a plurality of values of the hole diameter D corresponding to each through-hole 112. Therefore, in the present embodiment, in calculating the above-described ratio value, an arithmetic average value of two or more values of the hole diameter D corresponding to each of the plurality of through-holes 112 is used as a representative value of the hole diameter D. On the other hand, the pitch P of the plurality of through-holes 112 means a shortest distance between the opening portions of the two through-holes 112. However, with regard to the value of the pitch P, there are a plurality of values of the pitch P corresponding to a combination of the through-holes 112 adjacent to each other. Therefore, according to the present embodiment, in calculating the above-described ratio value, the arithmetic average value of two or more values of the pitch P corresponding to each combination of the through-holes 112 adjacent to each other is used as a representative value of the pitch P.

The pitch P of the above-described through-holes 112, the hole diameter D, and the ratio of the hole diameter D to the pitch P are merely examples, and the present invention is not limited thereto.

The adhesion promotion portion 110A can be made of a biodegradable material. The constituent material of the adhesion promotion portion 110A is not particularly limited, and examples thereof include a biodegradable resin. As the biodegradable resin, for example, it is possible to use a known biodegradable (co)polymer such as those disclosed in JP-T-2011-528275, JP-T-2008-514719, Pamphlet of International Publication No. 2008-1952, and JP-T-2004-509205. Specifically, the biodegradable resin includes (1) a polymer selected from a group formed of aliphatic polyester, polyester, polyanhydride, polyorthoester, polycarbonate, polyphosphazene, polyphosphate ester, polyvinyl alcohol, polypeptide, polysaccharide, protein, and cellulose; or (2) copolymer formed of one or more monomers forming the above-described materials (1). That is, it is preferable that the biodegradable sheet includes at least one biodegradable resin selected from a group formed of the polymer selected from a group formed of aliphatic polyester, polyester, polyanhydride, polyorthoester, polycarbonate, polyphosphazene, polyphosphate ester, polyvinyl alcohol, polypeptide, polysaccharide, protein, and cellulose, and the copolymer formed of one or more monomers forming the polymer.

A manufacturing method of the adhesion promotion portion 110A is not particularly limited. For example, the manufacturing method includes a method of preparing a fiber formed of the above-described biodegradable resin and manufacturing a mesh-shaped sheet by using the fiber. A method of preparing the fiber formed of the biodegradable resin is not particularly limited. For example, the method includes an electrospinning method (electric field spinning method and electrostatic spinning method) or a melt blowing method. As the method for the adhesion promotion portion 110A, only one of the above-described methods may be selected and used. Alternatively, two or more methods may be selected in appropriate combination with each other. As still another example of the manufacturing method of the adhesion promotion portion 110A, a fiber formed of the above-described biodegradable resin may be spun in accordance with a usual method, and the obtained fiber may be knitted into a mesh shape to manufacture the biodegradable sheet according to the present invention.

The adhesion promotion portion 110A causes a biological reaction by using the constituent materials such as the biodegradable resin constituting the adhesion promotion portion 110A. Due to this action, the adhesion promotion portion 110A induces expression of biological components such as fibrin. The biological components induced in this manner can promote adhesion by being accumulated to penetrate the through-holes 112 of the adhesion promotion portion 110A. Therefore, the adhesion promotion portion 110A is disposed between the biological organs to be joined, thereby promoting the adhesion by using the above-described mechanism.

The material of the adhesion promotion portion 110A may not be biodegradable as long as it is possible to promote the adhesion of the biological organs. In addition, the adhesion promotion portion 110A may not have the through-hole 112 regardless of the material, as long as it is possible to promote the adhesion of the biological organs.

### <Frame Portion 110B >

As illustrated in Fig. 1A, the frame portion 110B is formed on the adhesion promotion sheet 110 so as to surround the periphery of the adhesion promotion portion 110A. The frame portion 110B is preferably formed to have a higher rigidity than that of the adhesion promotion portion 110A so that the frame portion 110B is not easily deformed when an external force is applied. The frame portion 110B can be made of, for example, a biodegradable sheet in which a hole portion such as a through-hole 112 is not formed, a resin sheet having a higher rigidity than that of the adhesion promotion portion 110A, or a non-woven fabric.

In addition, the through-hole 112 is not formed in a certain region including the outer peripheral edge O of the biodegradable sheet which is a constituent material of the adhesion promotion portion 110A, so that the adhesion promotion sheet 110 may be provided with the frame portion 110B. In addition, after forming the through-hole 112 in a certain region including the outer peripheral edge O of the biodegradable sheet which is a constituent material of the adhesion promotion portion 110A, only the region is compressed or heated in the thickness direction to crush the through-hole 112. Accordingly, a portion in which the constituent materials of the biodegradable sheet are densely assembled may be formed, and the portion may be used as the frame portion 110B.

In addition, the frame portion 110B may be provided with a suppressing portion that suppresses a synechia with the biological organs at least in a part thereof. The material constituting the suppressing portion is not particularly limited as long as it is possible to suppress the synechia with the biological organs. For example, a non-woven fabric may be used. In addition, the suppressing portion can be made of a biodegradable material, similarly to the adhesion promotion portion 110A.

In the adhesion promotion sheet 110, the entire adhesion promotion sheet 110 may be configured to include the adhesion promotion portion 110A. That is, the adhesion promotion sheet 110 may not be provided with the frame portion 110B.

### <Holding Portion 120>

The holding portion 120 is disposed in the frame portion 110B of the adhesion promotion sheet 110.

The holding portion 120 includes one end portion 121a, the other end portion 121b, and an intermediate portion 122 extending between the one end portion 121a and the other end portion 121b. In the present embodiment, the holding portion 120 has a substantially C-shaped planar shape. The holding portion 120 is disposed only in a portion of the adhesion promotion sheet 110 along the circumferential direction.

The holding portion 120 is integrally attached to the adhesion promotion sheet 110. For example, the holding portion 120 can be disposed and fixed on the front surface 113 and the rear surface 114 of the adhesion promotion sheet 110, or inside the adhesion promotion sheet 110. For example, fixing can be configured by the adhesive or integrally molding the holding portion 120 when molding the adhesion promotion sheet 110.

The holding portion 120 can be configured to hold the adhesion promotion sheet 110 on the pancreatic parenchyma B1 by an elastic force. The material constituting the holding portion 120 is not particularly limited, and can be made of, for example, a superelastic alloy such as an alloy of titanium and nickel. However, other metal materials or resin materials that can impart elastic force may be used.

In addition, the holding portion 120 may be configured to be separable from, for example, the adhesion promotion sheet 110. In this configuration, the holding portion 120 is attached from the outer surface of the adhesion promotion sheet 110 in a state where the adhesion promotion sheet 110 is disposed so as to cover a portion of the pancreatic parenchyma B1. Accordingly, the adhesion promotion sheet 110 can be held on the pancreatic parenchyma B1 by the holding portion 120.

In addition, the shape and size of the holding portion 120, the position to be attached to the adhesion promotion sheet 110, and the like are not particularly limited.

### <Pulling Unit 140>

As illustrated in Fig. 1A, the medical device 100 includes a pulling unit 140 that enables a pulling operation of the adhesion promotion sheet 110 toward the side facing the position where the holding portion 120 is disposed. The "side facing the position where the holding portion 120 is disposed" is a position facing the holding portion 120 with the central portion C interposed therebetween in the adhesion promotion sheet 110.

The pulling unit 140 can include a string-shaped member or a strip-shaped member connected to the adhesion promotion sheet 110. The strip-shaped member has a certain width, and examples thereof include a member having a long side and a short side formed in a cross-sectional shape. In the present embodiment, a string-shaped member having a predetermined length is connected to the frame portion 110B. Although Fig. 1A illustrates a form in which a portion of the pulling unit 140 is disposed close to the holding portion 120, the specific arrangement form of the pulling unit 140 is not particularly limited.

For example, the pulling unit 140 can be made of a thermoplastic elastomer such as vinyl chloride, polyurethane elastomer, polystyrene elastomer, styrene-ethylene-butylene-styrene copolymer (SEBS), and styrene-ethylene-propylene-styrene copolymer (SEPS), a thermoplastic resin such as nylon and PET, or rubber, silicone elastomer, fiber material, and metals such as SUS wire, copper wire, titanium wire, and nitinol wire. In addition, the pulling unit 140 may be made of, for example, the same material as that of the adhesion promotion portion 110A. By using the same material as that of the adhesion promotion portion 110A, it is possible to manufacture at the same manufacturing site as that of the adhesion promotion portion 110A, so that the manufacturing work is easy.

The installation of the pulling unit 140 may be omitted. In addition, the position where the pulling unit 140 is disposed on the adhesion promotion sheet 110 is not particularly limited. In addition, the length, cross-sectional shape, thickness, number of installations, and the like of the pulling unit 140 are not particularly limited.

Fig. 1B illustrates a front view (front view viewed from the cut surface B1a of the pancreatic parenchyma B1) when the adhesion promotion sheet 110 is hooked and held on the pancreatic parenchyma B1 by the holding portion 120. For example, the operator can dispose the holding portion 120 so as to be hooked on the posterior wall B1c of the pancreatic parenchyma B1 (portion of the pancreatic parenchyma B1 on a dorsal side in the circumferential direction). As a result, the adhesion promotion portion 110A can be held on the pancreatic parenchyma B1 while preventing wrinkles and the like from being generated on the adhesion promotion sheet 110. In particular, in a case where the holding portion 120 is made of a material capable of imparting elastic force, by disposing the holding portion 120 so as to be fitted to the pancreatic parenchyma B1, it is possible to more reliably prevent the adhesion promotion sheet 110 from falling off. In addition, the operator can stretch the adhesion promotion sheet 110 by pulling the pulling unit 140 on the anterior wall B1d (portion of the pancreatic parenchyma B1 on a ventral side in the circumferential direction) side of the pancreatic parenchyma B1. As a result, it is possible to prevent wrinkles from being generated on the adhesion promotion sheet 110. By connecting the pulling unit 140 on the anterior wall B1d side of the pancreatic parenchyma B1, the operator can maintain the state where the pulling unit 140 applies the pulling force to the adhesion promotion sheet 110 even when the operator releases the fingers from the pulling unit 140.

As described above, the medical device 100 according to the present embodiment includes the adhesion promotion sheet 110 provided with the adhesion promotion portion 110A promoting the adhesion of the biological tissues and the holding portion 120 that allows the adhesion promotion sheet 110 to be hooked and held on the biological organ to be joined.

According to the medical device 100 configured as described above, the adhesion of the biological tissues of the biological organs can be promoted by interposing the adhesion promotion sheet 110 between the joint target sites of the biological organs to be joined. In addition, the operator can hook and hold the adhesion promotion sheet 110 on a portion of the biological organ (for example, pancreatic parenchyma B1) to be joined by the holding portion 120. Therefore, the operator can prevent the adhesion promotion sheet 110 from falling off from the biological organs during a medical procedure. Therefore, the risk of anastomotic leakage of the biological organs can be effectively reduced.

In addition, the holding portion 120 is configured to be able to hold the adhesion promotion sheet 110 on the biological organ by elastic force. Therefore, the operator can more reliably hold the adhesion promotion sheet 110 on the biological organs by the elastic force of the holding portion 120.

In addition, the holding portion 120 is integrally attached to the adhesion promotion sheet 110. Therefore, it is possible to prevent the holding portion 120 from being separated from the adhesion promotion sheet 110, and to maintain the state where the adhesion promotion sheet 110 is held on the biological organs more stably.

In addition, the holding portion 120 is disposed only in a portion of the adhesion promotion sheet 110 along the circumferential direction. Therefore, the holding portion 120 is disposed so as to be hooked on a portion of the outer peripheral surface of the biological organ (for example, a portion of the posterior wall B1c of the pancreatic parenchyma B1). Accordingly, the adhesion promotion portion 110A can be appropriately disposed on the biological organ, while preventing wrinkles or the like from being generated on the adhesion promotion sheet 110.

The medical device 100 includes the pulling unit 140 that allows the pulling operation of the adhesion promotion sheet 110 toward the side facing the position where the holding portion 120 is disposed. Therefore, the operator can more reliably prevent wrinkles from being generated on the adhesion promotion sheet 110 by pulling the pulling unit 140.

In addition, the pulling unit 140 can include a string-shaped member or a strip-shaped member connected to the adhesion promotion sheet 110. The operator can prevent wrinkles from being generated on the adhesion promotion sheet 110 by pulling the pulling unit 140 by grasping the pulling unit 140 with fingers or the like.

In addition, the frame portion 110B can prevent the frame portion 110B from performing the synechia with the biological organs other than the biological organs to be joined by the suppressing portion that suppresses the synechia with the biological organs.

Next, a modification example of the above-described embodiment will be described. In the description of the modification example, detailed description of the constituent members and the like already described in the above-described embodiment will be omitted. In addition, the contents not particularly described in the description of the modification example can be the same as those in the above-described embodiment.

<Modification example 1>

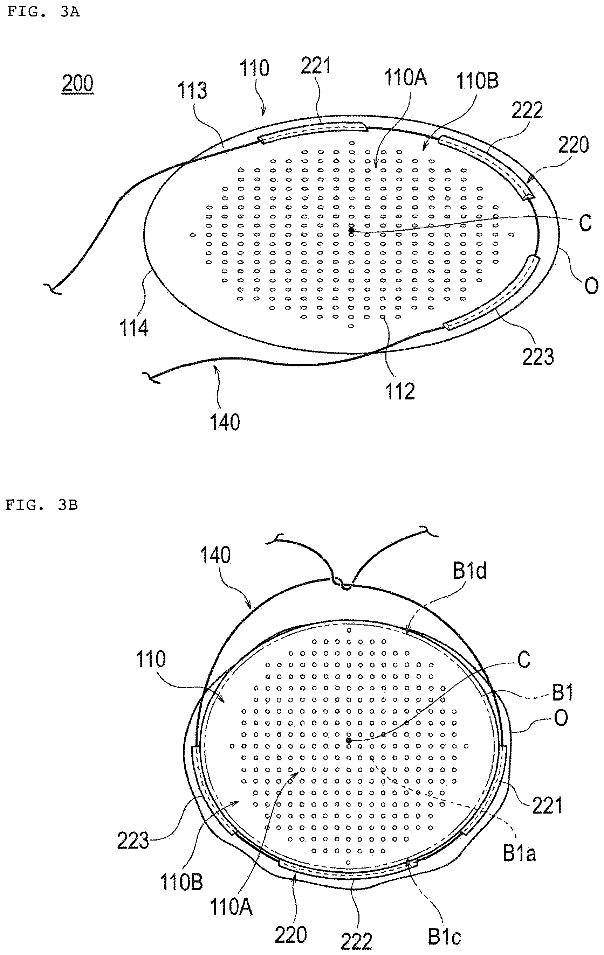

Fig. 3A is a perspective view of a medical device 200 according to Modification Example 1, and Fig. 3B is a diagram for describing a usage example of the medical device 200 according to Modification Example 1.

The holding portion 220 may not include, for example, one continuous member. In the medical device 200 according to Modification Example 1, as illustrated in Figs. 3A and 3B, the holding portion 220 is configured to include divided pieces 221, 222, and 223 divided into three in the arrangement direction of the holding portion 220 (circumferential direction of the adhesion promotion sheet 110). As illustrated in Fig. 3B, the holding portion 220 according to Modification Example 1 exhibits a function of holding the adhesion promotion sheet 110 on the biological organs, similarly to the holding portion 120 according to the above-described embodiment. In a case where the holding portion 220 is configured to include a plurality of divided pieces, the number of divided pieces, the length, shape, material, and the like of each divided piece are not particularly limited.

### <Modification Example 2>

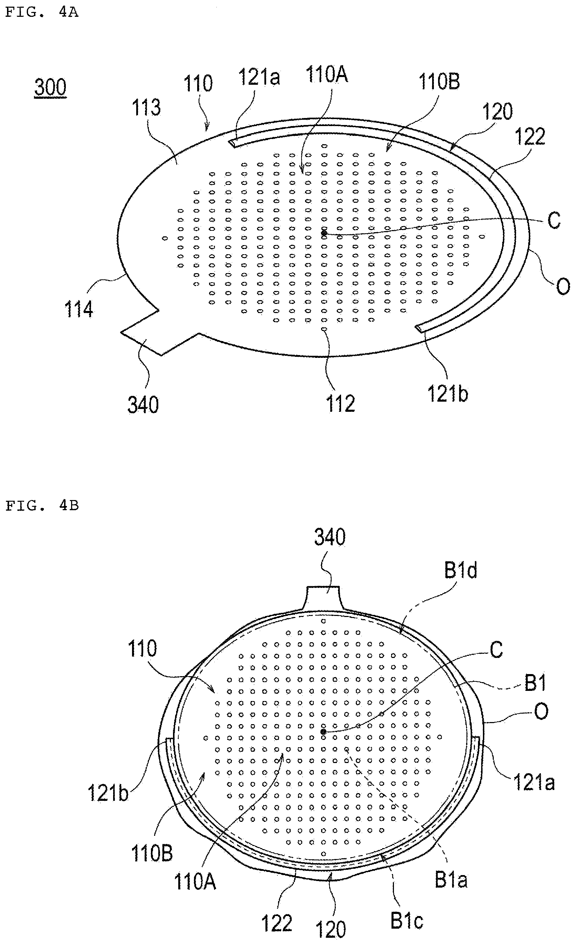

Fig. 4A is a perspective view of a medical device 300 according to Modification Example 2, and Fig. 4B is a diagram for describing a usage example of the medical device 300 according to Modification Example 2.

The pulling unit 340 may be configured to include, for example, a portion of the adhesion promotion sheet 110. In the medical device 300 according to Modification Example 2, a pulling unit 340 is configured to include a projection portion on which a portion of the adhesion promotion sheet 110 projects. As illustrated in Fig. 4B, when the adhesion promotion sheet 110 is held on the pancreatic parenchyma B1, the pulling unit 340 is lifted to the side facing the side where the holding portion 120 is provided (upper side in the drawing). Accordingly, it is possible to prevent wrinkles from being generated on the adhesion promotion sheet 110. The specific shape, size, number, and the like of the pulling unit 340 are not particularly limited.

### <Modification Example 3>

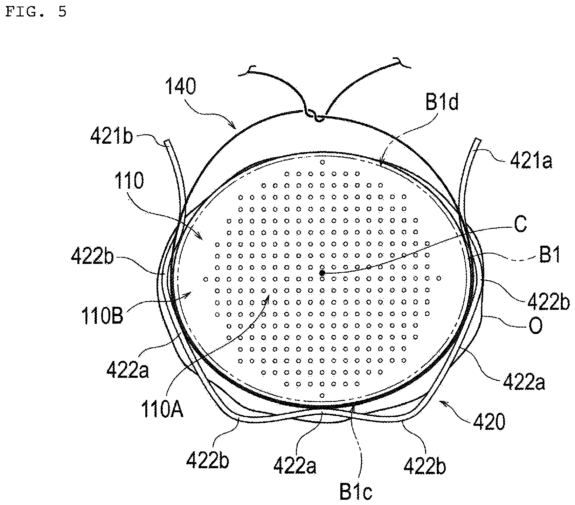

Fig. 5 is a diagram for describing a usage example of a medical device according to Modification Example 3.

In the medical device according to Modification Example 3, the shape of a holding portion 420 is different from that of the holding portion 120 of the above-described embodiment. As illustrated in Fig. 5, the holding portion 420 includes a plurality of first sites 422a that are disposed so as to come into contact with the pancreatic parenchyma B1 (indirect contact with the adhesion promotion sheet 110 interposed therebetween) when hooked on the pancreatic parenchyma B1 and a plurality of second sites 422b disposed with a gap between the plurality of second sites 422b and the pancreatic parenchyma B1. The first site 422a and the second site 422b are alternately disposed in the circumferential direction of the adhesion promotion sheet 110. The holding portion 420 can reduce the area in contact with the pancreatic parenchyma B1 as compared with the holding portion 120 formed in the C shape (refer to Fig. 1B) described above. Therefore, it is possible to reduce the burden on the patient while the adhesion promotion sheet 110 is held on the pancreatic parenchyma B1 by the holding portion 420.

### <Embodiment of Treatment Method (Biological Organs Anastomosis)>

Next, a treatment method using a medical device will be described.

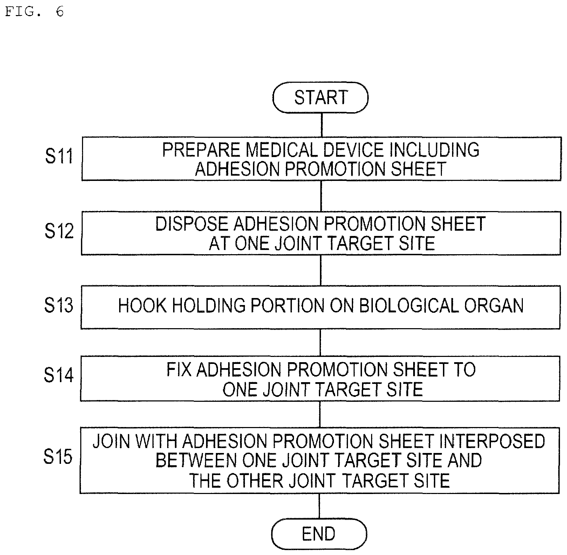

Fig. 6 is a flowchart illustrating each procedure of the treatment method using the medical device.

The treatment method includes preparing a medical device including an adhesion promotion sheet provided with a holding portion (S11), disposing the adhesion promotion sheet at one joint target site (S12), hooking the holding portion on the joint target site (S13), fixing the adhesion promotion sheet to the one joint target site (S14), and joining the one joint target site and the other joint target site in a state where at least a portion of the adhesion promotion sheet is disposed between the one joint target site and the other joint target site (S15).

The biological organs and the joint target site in the biological organs which are joined by using the treatment method are not particularly limited, and can be optionally selected. In the following description, pancreatic parenchyma-jejunum anastomosis will be described as an example. However, the above-described treatment method may be applied to large intestine anastomosis or gastric tube anastomosis. In addition, as the medical device used in each medical procedure described below, for example, it is possible to select any desired one from the medical devices described above. However, in the following description, as a representative example which can be preferably used for each medical procedure, an example of using a specific medical device will be described. In addition, in each medical procedure described below, detailed description of known medical procedures, known medical devices, and medical instruments will be appropriately omitted.

Hereinafter, in the description herein, "disposing the adhesion promotion sheet between the biological organs" means at least any one of disposing the adhesion promotion sheet in a state of being in direct or indirect contact with the biological organs, disposing the adhesion promotion sheet in a state where a spatial gap is formed with the biological organs, and disposing the adhesion promotion sheet in both the states (for example, disposing the adhesion promotion sheet in a state where the adhesion promotion sheet is in contact with one biological organ and the adhesion promotion sheet is not in contact with the other biological organ). In addition, in the description herein, a "periphery" does not define a strict range (region), and means a predetermined range (region) as long as a treatment purpose (joining the biological organs to each other) can be achieved. In addition, as long as the treatment purpose can be achieved, in the medical procedure described in the respective treatment methods, orders can be appropriately switched thereamong.

### <Embodiment of Treatment Method (Pancreatic Parenchyma-Jejunum Anastomosis)>

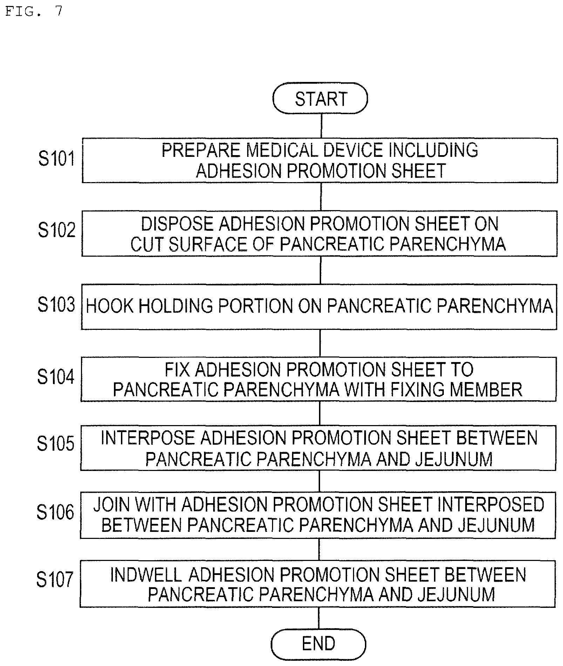

Fig. 7 is a flowchart illustrating a procedure of an embodiment of the treatment method (pancreatic parenchyma-jejunum anastomosis), and Figs. 8 to 15 are diagrams used for describing the pancreatic parenchyma-jejunum anastomosis.

In the treatment method according to the present embodiment, the biological organs to be joined are the pancreatic parenchyma B1 after pancreaticoduodenectomy and the jejunum B2. In the following description, a procedure of joining the periphery of the cut surface B1a of the cut pancreatic parenchyma B1 (one joint target site) and a predetermined site of an intestinal wall of the jejunum B2 (the other joint target site) will be described. In addition, in the present embodiment, the usage example of the medical device 100 illustrated in Fig. 1A will be described.

As illustrated in Fig. 7, the treatment method according to the present embodiment includes preparing the medical device 100 including the adhesion promotion sheet 110 provided with the holding portion 120 (S101), disposing the adhesion promotion sheet 110 on the cut surface B1a of the pancreatic parenchyma B1 (S102), hooking the holding portion 120 on the pancreatic parenchyma B1 (S103), fixing the adhesion promotion sheet 110 with a fixing member (S104), interposing the adhesion promotion sheet 110 between the pancreatic parenchyma B1 and the jejunum B2 (S105), joining with the adhesion promotion sheet 110 interposed between the pancreatic parenchyma B1 and the jejunum B2 (S106), and indwelling the adhesion promotion sheet 110 between the pancreatic parenchyma B1 and the jejunum B2 (S107).

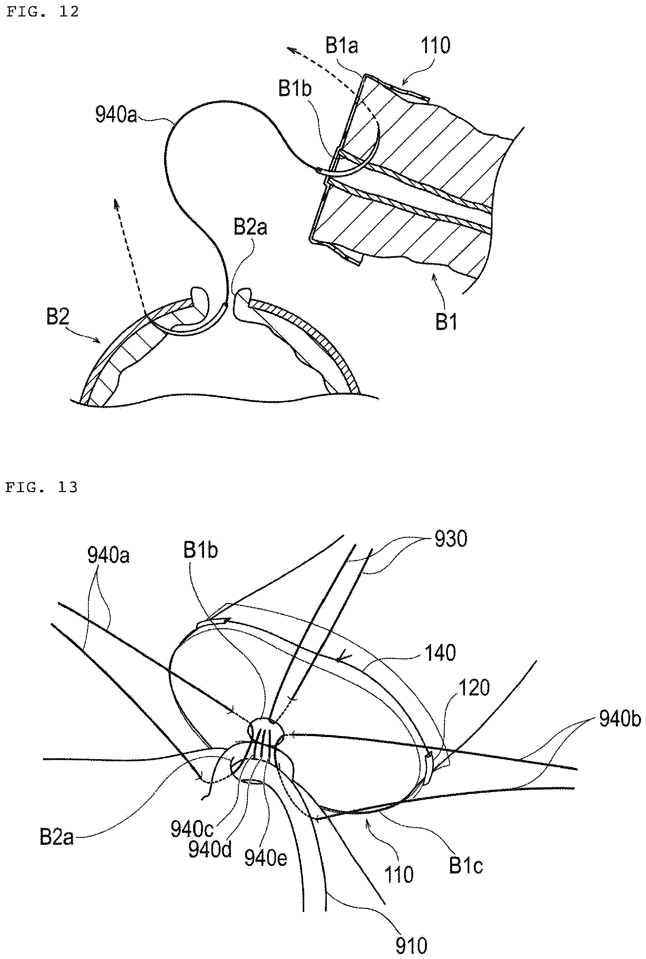

Next, an example of the treatment method according to the present embodiment will be specifically described with reference to Figs. 8 to 15. In Fig. 13, a plurality of both end needles 920a to 920e described later are omitted.

As illustrated in Fig. 8, the operator causes the rear surface 114 (or front surface 113) of the adhesion promotion sheet 110 face the cut surface B1a of the pancreatic parenchyma B1. The operator disposes the holding portion 120 so as to be located outer side than the cut surface B1a in the plane direction. At this time, the operator can hold the adhesion promotion sheet 110 on the pancreatic parenchyma B1 by disposing the holding portion 120 so as to be hooked on the posterior wall B1c of the pancreatic parenchyma B1 (portion of the pancreatic parenchyma B1 on a dorsal side in the circumferential direction). In addition, by pulling the pulling unit 140, the adhesion promotion portion 110A can be brought into close contact with the cut surface B1a of the pancreatic parenchyma B1 while preventing wrinkles from being generated on the adhesion promotion sheet 110 (refer to Fig. 1B) .

When disposing the adhesion promotion sheet 110 on the cut surface B1a of the pancreatic parenchyma B1, the operator can adopt the following work procedure. First, the operator forms a hole portion 130 in the adhesion promotion sheet 110 by pressing an end portion 911 (or end portion 912) of a pancreatic duct tube 910 against the adhesion promotion sheet 110. In addition, the operator inserts the pancreatic duct tube 910 into the jejunum B2 so that the end portion 911 of the pancreatic duct tube 910 passes through the inside of the jejunum B2 from the through-hole B2a at the planned anastomosis site of jejunum B2 and exits the outside of the jejunum B2 from the through-hole B2b of the jejunum B2.

Next, the operator temporarily inserts the end portion 912 of the pancreatic duct tube 910 into the pancreatic duct B1b of the pancreatic parenchyma B1 in a state where the pancreatic duct tube 910 inserts the hole portion 130 of the adhesion promotion sheet 110 and holds the adhesion promotion sheet 110.

As the pancreatic duct tube 910, for example, a known resin tube in which a bump (projection portion) for preventing falling off is formed at the end portion 912 can be used. The pancreatic duct tube 910 temporarily inserted into the pancreatic duct B1b suppresses the leakage of body fluid such as pancreatic juice from the pancreatic duct B1b during the medical procedure. According to such a procedure, the operator can dispose the adhesion promotion sheet 110 and temporarily insert the pancreatic duct tube 910 at the same time.

In addition, the operator may use a device other than the pancreatic duct tube 910 when forming the hole portion 130 for inserting the pancreatic duct tube 910. In addition, the hole portion 130 through which the pancreatic duct tube 910 is inserted may be formed in the adhesion promotion sheet 110 in advance in a state before use. In addition, the operator may temporarily insert the pancreatic duct tube 910 into the pancreatic duct B1b after disposing the adhesion promotion sheet 110 on the cut surface B1a of the pancreatic parenchyma B1.

Next, the operator fixes the adhesion promotion sheet 110 to the pancreatic parenchyma B1 with the fixing member. In the following description, an example of a procedure of fixing the adhesion promotion sheet 110 to the pancreatic parenchyma B1 by using the plurality of the both end needles 920a to 920e with sutures as fixing members will be described. As the both end needles 920a to 920e, known needles having a bioabsorbable absorbent thread (suture) and a biocompatible needle portion attached to both ends of the absorbent thread can be used. Both end needles 930 and 940a to 940e described later are also configured to include absorbent threads and needle portions.

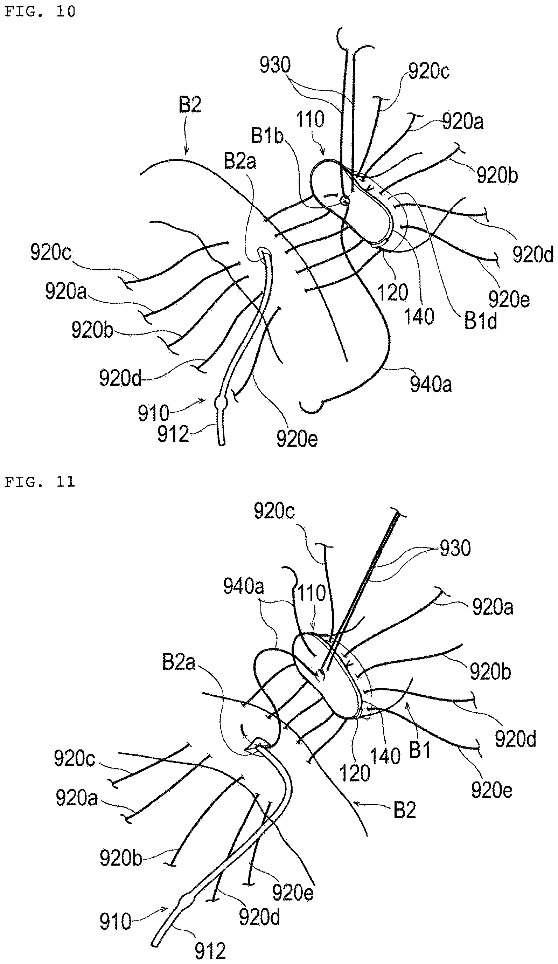

First, as illustrated in Fig. 9, the operator moves the both end needle 920a from the posterior wall B1c of the pancreatic parenchyma B1 (portion of the pancreatic parenchyma B1 on a dorsal side in the circumferential direction) and the portion disposed on the posterior wall B1c in the adhesion promotion sheet 110 toward the anterior wall B1d of the pancreatic parenchyma B1 and the portion disposed on the anterior wall B1d in the adhesion promotion sheet 110 in a state where the adhesion promotion sheet 110 is held on the pancreatic parenchyma B1. Next, the operator moves the both end needle 920a so as to insert a jejunal serosal muscular layer at the planned anastomosis site of the jejunum B2 (periphery of the through-hole B2a). The operator repeats such an operation, and as illustrated in Fig. 10, inserts the plurality of the both end needles 920a to 920e into the plurality of the both end needles 920a to 920e on the adhesion promotion sheet 110, the pancreatic parenchyma B1, and the jejunal serosal muscular layer of the jejunum B2. In this manner, the operator can fix the adhesion promotion sheet 110 to the pancreatic parenchyma B1 by using the plurality of the both end needles 920a to 920e that suture the pancreatic parenchyma B1 and the jejunum B2.

The operator may interrupt the holding by the holding portion 120 and the pulling operation by the pulling unit 140 after fixing the adhesion promotion sheet 110 to the cut surface B1a of the pancreatic parenchyma B1. The holding portion 120 and the pulling unit 140 may be appropriately separated from the adhesion promotion sheet 110 by cutting off a portion of the adhesion promotion sheet 110, or the like. The operator performs holding by the holding portion 120 and pulling by the pulling unit 140 until the adhesion promotion sheet 110 is fixed to the cut surface B1a of the pancreatic parenchyma B1, so that it is possible to prevent the adhesion promotion sheet 110 from being misaligned from the cut surface B1a of the pancreatic parenchyma B1 or wrinkles from being generated on the adhesion promotion sheet 110.

The number of both end needles to be inserted into the pancreatic parenchyma B1 and the jejunal serosal muscular layer of the jejunum B2 and the positions through which the both end needles are inserted are not particularly limited. In addition, the operator may fix the adhesion promotion sheet 110 to the pancreatic parenchyma B1 by using a biodegradable stapler or the like as a fixing member instead of the plurality of the both end needles 920a to 920e.

Next, as illustrated in Fig. 10, the operator removes the end portion 912 of the pancreatic duct tube 910 from the pancreatic duct B1b.

Next, as illustrated in Fig. 10, the operator passes the both end needle 930 from a luminal side of the pancreatic duct B1b toward the anterior wall B1d side of the cut surface B1a of the pancreatic parenchyma B1. The both end needle 930 is held by a gripping instrument such as tweezers (not illustrated) so as not to interfere with the medical procedure in a state where the jejunum B2 is not inserted.

Next, as illustrated in Figs. 10 and 12, the operator moves one end of the both end needle 940a from the luminal side of the pancreatic duct B1b toward the cut surface B1a of the pancreatic parenchyma B1. Next, as illustrated in Figs. 11 and 12, the operator inserts the other end of the both end needle 940a into the through-hole B2a of the jejunum B2, and moves the other end of the both end needle 940a from the inside of the jejunum B2 toward the outside of the jejunum B2. As illustrated in Fig. 13, the operator inserts the plurality of both end needles 940a to 940e into different sites of the pancreatic duct B1b in the circumferential direction and the jejunum B2. Fig. 12 is a cross-sectional view schematically illustrating a portion of the pancreatic parenchyma B1 and the jejunum B2 before being anastomosed.

Next, as illustrated in Fig. 13, the operator brings the posterior wall B1c of the pancreatic parenchyma B1 and the pancreatic duct B1b into close contact with the planned anastomosis site of the jejunum B2. Of the plurality of the both end needles 940a to 940e, the both end needles 940c to 940e that insert the dorsal side (posterior wall B1c side) of the pancreatic duct B1b in the circumferential direction are ligated.

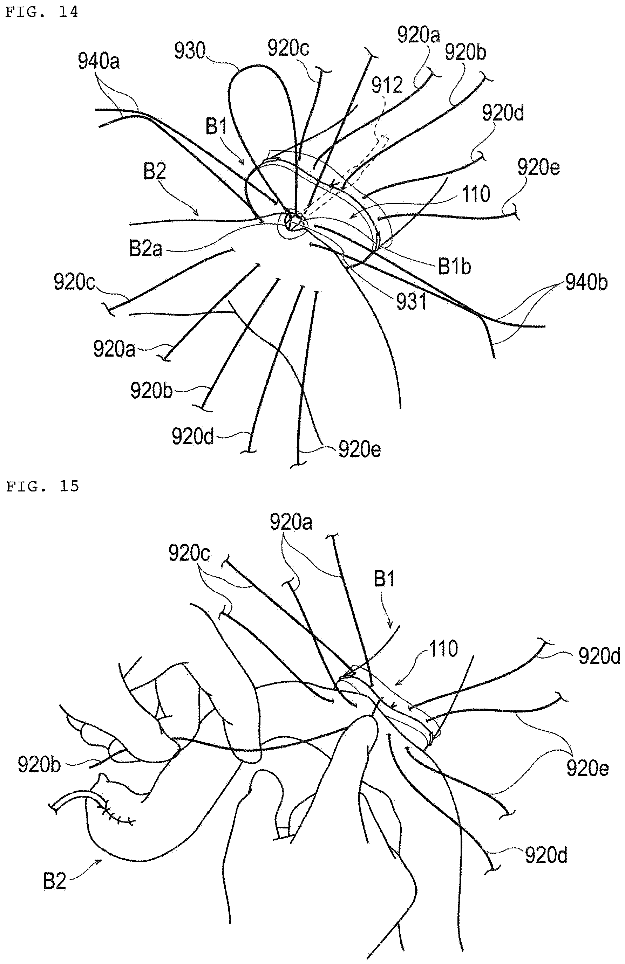

Next, as illustrated in Fig. 14, the operator reinserts the end portion 912 of the pancreatic duct tube 910 into the pancreatic duct B1b. Next, the operator inserts a needle portion 931 extending from the inside of the pancreatic duct B1b in the both end needle 930 into the through-hole B2b formed in the jejunum B2, and moves the needle portion 931 from the inside of the jejunum B2 toward the outside of the jejunum B2.

Next, the operator ligates the both end needles 930, 940a, and 940b (not illustrated). The number of both end needles to be inserted into the pancreatic duct B1b and the jejunum B2 and the positions through which the both end needles are inserted are not particularly limited.

Next, as illustrated in Fig. 15, the operator ligates the both end needles 920a to 920e while pressing the jejunum B2 against the pancreatic parenchyma B1 with the operator's finger. As a result, the pancreatic parenchyma B1 and the jejunum B2 are sutured in a state where the adhesion promotion sheet 110 is interposed therebetween. The jejunum B2 is deformed by the tension generated at the time of suturing so as to enclose the cut surface B1a of the pancreatic parenchyma B1 and the adhesion promotion portion 110A of the adhesion promotion sheet 110.

The operator indwells the adhesion promotion sheet 110 in a state where the adhesion promotion portion 110A of the adhesion promotion sheet 110 is interposed between the cut surface B1a of the pancreatic parenchyma B1 and the intestinal wall of the jejunum B2. The adhesion promotion portion 110A of the adhesion promotion sheet 110 is indwelled between the cut surface B1a of the pancreatic parenchyma B1 and the intestinal wall of the jejunum B2 while being in contact with the cut surface B1a of the pancreatic parenchyma B1 and the intestinal wall of the jejunum B2. Accordingly, the adhesion of the biological tissue of the pancreatic parenchyma B1 and the biological tissue of the intestinal wall of the jejunum B2 is promoted.

As described above, the treatment method according to the present embodiment is applied to the medical procedure of joining the pancreatic parenchyma B1 and the jejunum B2. In addition, in the above treatment method, the periphery of the cut surface B1a of the cut pancreatic parenchyma B1 and the intestinal wall (jejunal serosal muscular layer) of the jejunum B2 are joined to each other. According to the treatment method, the adhesion promotion portion 110A of the adhesion promotion sheet 110 interposed between the cut surface B1a of the pancreatic parenchyma B1 and the intestinal wall of the jejunum B2 can promote the adhesion of the biological tissue of the pancreatic parenchyma B1 and the biological tissue of the intestinal wall of the jejunum B2, and can reduce the risk of anastomotic leakage after the pancreatic parenchyma-jejunum anastomosis.

In addition, by holding the adhesion promotion sheet 110 on the pancreatic parenchyma B1 by the holding portion 120, it is possible to prevent the adhesion promotion sheet 110 from misaligning or falling off from the pancreatic parenchyma B1 during the medical procedure. In addition, by pulling the pulling unit 140 to apply the pulling force to the adhesion promotion sheet 110, it is possible to hold the adhesion promotion sheet 110 on the pancreatic parenchyma B1 while preventing wrinkles from being generated on the adhesion promotion sheet 110.

This application is based on Japanese Patent Application No. 2019-065058 filed on March 28, 2019, the disclosure content of which is incorporated by reference in its entirety.

### Reference Signs List

100, 200, 300: medical device,
110: adhesion promotion sheet,
110A: adhesion promotion portion,
110B: frame portion,
112: through-hole,
120, 220, 420: holding portion,
140, 340: pulling unit,
B1: pancreatic parenchyma,
B2: jejunum.

## Claims

1. A medical device comprising:
an adhesion promotion sheet configured to include an adhesion promotion portion promoting adhesion of biological tissues; and
a holding portion configured to hook and hold the adhesion promotion sheet on a biological organ to be joined.

2. The medical device according to Claim 1,
wherein the holding portion is configured to hold the adhesion promotion sheet on the biological organ by elastic force.

3. The medical device according to Claim 1 or 2,
wherein the holding portion is integrally attached to the adhesion promotion sheet.

4. The medical device according to any one of Claims 1 to 3,
wherein the holding portion is disposed only in a portion of the adhesion promotion sheet along a circumferential direction.

5. The medical device according to any one of Claims 1 to 4, further comprising:
a pulling unit configured to perform a pulling operation of the adhesion promotion sheet toward a side facing a position where the holding portion is disposed.

6. The medical device according to Claim 5,
wherein the pulling unit is a string-shaped member or a strip-shaped member connected to the adhesion promotion sheet.
